Europäisches Patentamt

**European Patent Office**

**Office européen des brevets**

(19)

(11) Veröffentlichungsnummer : **0 121 684
B1**

(12) **EUROPÄISCHE PATENTSCHRIFT**

(45) Veröffentlichungstag der Patentschrift :
04.03.87

(21) Anmeldenummer : **84101462.4**

(22) Anmeldetag : **13.02.84**

(51) Int. Cl.⁴ : **C 07 C 63/70, C 07 C 51/265**

(54) Verfahren zur Herstellung von Halogenbenzoesäuren aus im Kern halogenierten Toluolen.

(30) Priorität : **10.03.83 DE 3308448**

(43) Veröffentlichungstag der Anmeldung :
**17.10.84 Patentblatt 84/42**

(45) Bekanntmachung des Hinweises auf die Patenterteilung : **04.03.87 Patentblatt 87/10**

(84) Benannte Vertragsstaaten :
**DE GB IT NL**

(56) Entgegenhaltungen :
**EP-A- 0 070 393**

(73) Patentinhaber : **DYNAMIT NOBEL AKTIENGESELLS-
CHAFT
Postfach 1209
D-5210 Troisdorf, Bez. Köln (DE)**

(72) Erfinder : **Feld, Marcel, Dr.
Im Lochgarten 56
D-5000 Köln 90 (DE)**

**Beschreibung**

Die vorliegende Erfindung betrifft ein Verfahren zur Herstellung von Halogenbenzoesäuren der im Oberbegriff von Patentanspruch 1 bezeichneten Art.

Die als Ausgangsmaterialien eingesetzten im Kern halogenierten Toluole können ein oder mehrere, gleiche oder unterschiedliche Halogenatome tragen. Die daraus gewonnenen Halogenbenzoesäuren, wie z. B. o- oder p-Chlorbenzoesäure, können beispielsweise als Ausgangsprodukte für die Herstellung von Arzneimitteln, Schädlingsbekämpfungsmitteln und Farbstoffen dienen.

Die Oxidation mit Sauerstoff von im Kern halogenierten Toluolen zu den entsprechenden Halogenbenzoesäuren in flüssiger Phase ist in der Literatur bereits beschrieben. In einer kinetischen Untersuchung der Oxidation von Chlortoluolen (Zh. Prikl. Khim. (Leningrad) 1977, 50 (1), 133-6 ; Chem. Abstr. 87 : 22684 z) werden Essigsäure als Lösungsmittel, Kobaltacetat als Katalysator und Natriumbromid als Cokatalysator für die mit reinem Sauerstoff durchgeführte Reaktion verwendet. Dabei blieben jedoch technische Probleme, wie beispielsweise Korrosionswirkungen, oder wirtschaftliche Aspekte bezüglich der Produktisolierung außer Betracht. Das gilt auch für das in der EP-OS 0 002 749 beschriebene Oxidationsverfahren, das u. a. die Herstellung von Halogenbenzoesäuren betrifft. Es ist durch die Verwendung relativ geringer Mengen aliphatischer Carbonsäuren als Lösungsmittel gekennzeichnet.

Als vorteilhaft werden pro Gewichtsteil des zu oxidierenden Alkylaromaten weniger als 0,8 Gew.-Teile des Lösungsmittels, beispielsweise Essigsäure, genannt. Höhere Lösungsmittelkonzentrationen im Einsatzgemisch werden als nachteilig bezüglich Umsatz und Selektivität der Oxidation bezeichnet. Bei Verwendung größerer Lösungsmittelmengen sinkt die Ausbeute an aromatischer Carbonsäure, wohingegen der Anteil des als Zwischenprodukt gebildeten aromatischen Aldehyds im Reaktionsgemisch steigt.

Ein Nachteil größerer Lösungsmittelmengen wird in höheren Lösungsmittelverlusten und ferner, bedingt durch die Löslichkeit des Zielproduktes, in höheren Produktverlusten bzw. im Falle einer destillativen Aufarbeitung des Reaktionsgemisches in einem höheren Energieaufwand gesehen.

Die in der EP-OS 0 002 749 beanspruchten Lösungsmittelkonzentrationen haben nun bei der Herstellung bestimmter, im Kern halogenierter Benzoesäuren, wie beispielsweise der o-Chlor- oder p-Fluorbenzoesäure, zur Folge, daß das gesamte Reaktionsgemisch beim Abkühlen zu einer festen, nicht mehr fließfähigen Masse erstarrt. Die Isolierung des Zielproduktes kann dann nicht einfach durch Filtration des abgekühlten Reaktionsgemisches erfolgen. Es wird daher auch eine destillative Aufarbeitung der Reaktionsgemische in Betracht gezogen.

Eine destillative Aufarbeitung des gesamten Reaktionsgemisches kann zwar zu einem Zielprodukt hoher Reinheit führen, sie ist technisch und energetisch jedoch insbesondere dann relativ aufwendig, wenn es sich um Produkte mit Schmelzpunkten von weit über 100 °C handelt. Besonders schwierig wird es, wenn die Zielprodukte, wie beispielsweise o-Chlorbenzoesäure oder p-Fluorbenzoesäure, gar nicht destillierbar sind.

Erschwerend wirkt sich bei einer destillativen Aufarbeitung des Reaktionsgemisches auch noch die hohe, mit steigender Temperatur und sinkendem wäßrigen Verdünnungsgrad stark zunehmende Korrosivität einer bromidhaltigen Carbonsäure aus. Diese nachteilige Wirkung des für den Reaktionsverlauf so vorteilhaften Bromid-Cokatalysators erfordert besonders korrosionsfeste Materialien für alle jene Apparateteile, die mit dem Reaktionsgemisch bei hohen Temperaturen in Berührung kommen.

Das Korrosionsproblem wird im Falle der Oxidation von im Kern halogenierten Toluolen noch dadurch verschärft, daß durch eine teilweise oxidative Zerstörung von Ausgangs-, Zwischen- oder Endprodukten Halogenionen gebildet werden. So konnte beispielsweise bei der Oxidation von Chlortoluolen in der Mutterlauge nach Abtrennung des Zielproduktes neben dem als Cokatalysator eingesetzten Bromid auch noch Chlorid nachgewiesen werden. Selbst wenn diese unerwünschte Nebenreaktion durch geeignete Reaktionsbedingungen weitgehendst unterdrückt werden kann, können sich bei häufiger Wiederverwendung der Mutterlauge in dieser aus dem halogenierten Toluol stammende Halogenionen ansammeln.

Der vorliegenden Erfindung liegt die Aufgabe zugrunde, ein Verfahren zur Herstellung von im Kern halogenierten Benzoesäuren durch Oxidation der entsprechenden Toluole mit Sauerstoff bei starker Verringerung der Korrosivität des Reaktionsgemisches und der Mutterlauge zu entwickeln, das die Gewinnung der Zielprodukte in hoher Ausbeute und Reinheit ohne eine destillative Aufarbeitung des gesamten Reaktionsgemisches, d. h. ohne eine Destillation oder Sublimation des Zielproduktes ermöglicht. Die Isolierung der im Kern halogenierten Benzoesäure aus dem Reaktionsgemisch sollte vielmehr durch eine übliche Fest-Flüssig-Trennung erfolgen.

Gemäß der Erfindung wird diese Aufgabe dadurch gelöst, daß pro Gewichtsteil halogeniertem Toluol 1,5 bis 4,5 Gew.-Teil einer wäßrig-verdünnten Essigsäure mit 1 bis 35 Gew.-% Wasser und einem Gehalt an einer löslichen Ammonium- und/oder Alkali- und/oder Erdalkaliverbindung von mehr als 1,0 Äquivalenten pro Äquivalent einer im Reaktionsgemisch eingesetzten Bromidionen liefernden Verbindung verwendet werden.

Charakteristisch für das Verfahren ist die Verwendung einer wäßrig-verdünnten Essigsäure mit 1 bis ca. 35 Gew.-%, vorzugsweise 5 bis 25 Gew.-%, Wasser als Lösungsmittel, wobei pro Gewichtsteil des im Kern halogenierten Toluols mindestens 1,5 Gew.-Teile, vorzugsweise 2,0 bis 3,5 Gew.-Teile, des Lösungsmittels verwendet werden.

Ein weiteres Merkmal des Verfahrens ist ein Gehalt an Ammonium- und/oder Alkali- und/oder Erdalkaliionen von 1 oder mehr Äquivalenten pro Äquivalent Halogenionen im Reaktionsgemisch. Folge der erfindungsgemäßen Bedingungen und zugleich weiteres Merkmal einer bevorzugten Verfahrensweise ist eine häufige Wiederverwendung der nach Abtrennungen des Zielproduktes und des Reaktionswassers resultierenden Mutterlauge als Reaktionsmedium für weitere Oxidationen des betreffenden im Kern halogenierten Toluols.

Um die vorgesehene Produktisolierung durch eine Filtration zu ermöglichen, werden 1,5 bis 4,5 Gew.-Teile Lösungsmittel pro Gewichtsteil des zu oxidierenden halogenierten Toluols verwendet. Als Lösungsmittel dient dabei allerdings nicht reine Essigsäure, sondern eine wäßrigverdünnte Essigsäure mit einem Wassergehalt von 1 bis 35 Gew.-%, vorzugsweise 5 bis 25 Gew.-%, bzw. die nach Abtrennung des Zielproduktes mit einem entsprechenden Wassergehalt gewonnene Mutterlauge.

Das Ziel der hohen Produktausbeute wird durch den relativ hohen Wassergehalt im Reaktionsgemisch begünstigt. Im Falle in Essigsäure besonders gut löslicher Zielprodukte kann die Produktausbeute im Aufarbeitungsschritt dadurch weiter verbessert werden, daß dem noch heißen Reaktionsgemisch weiteres Wasser zugefügt wird, wodurch im Reaktionsgemisch ein Essigsäure/Wasser-Gewichtsverhältnis von 1 : 1 bis 3 : 1 eingestellt wird.

In einer bevorzugten Verfahrensweise wird eine hohe Ausbeute dadurch erreicht, daß die nach Abtrennung des Zielproduktes, des Reaktionswassers und der mit den Waschfiltraten und einer eventuellen nachträglichen Verdünnung des Reaktionsgemisches nach beendeter Reaktion zusätzlich eingebrachten Mengen von Wasser und Essigsäure erhaltene Mutterlauge mehrfach als Reaktionsmedium für weitere Oxidationen des betreffenden halogenierten Toluols wiederverwendet wird. Auf diese Weise werden allerdings nicht nur die lösungsbedingten Produktverluste insgesamt reduziert und dementsprechend die Ausbeute erhöht, sondern zugleich wird auch der Katalysatorbedarf beträchtlich gesenkt, da mit der Mutterlauge auch der Schwermetallkatalysator wiederverwendet wird. Lediglich die während der Oxidation eingetretenen Bromidverluste müssen in der rezyklisierten Mutterlauge ausgeglichen werden, was vorzugsweise durch entsprechende Zusätze von Bromwasserstoffsäure erfolgt.

Das Ziel der hohen Produktreinheit wird im allgemeinen durch einfaches Auswaschen des nach einem der üblichen Fest-Flüssig-Trennungen aus dem abgekühlten Reaktionsgemisch isolierten Zielproduktes mit wäßrig-verdünnter Essigsäure erreicht. Als Waschflüssigkeit dient dabei Essigsäure mit einem Wassergehalt von 10 bis 80 Gew.-%, vorzugsweise 30 bis 60 Gew.-%. Das Waschfiltrat kann mit der Mutterlauge zusammen destillativ aufgearbeitet werden. Auch ohne zusätzliche Reinigungsmaßnahme, etwa eine Umkristallisation oder Sublimation, wird nach dem erfindungsgemäßen Verfahren, selbst bei wiederholter Rückführung der Mutterlauge, z. B. o-Chlorbenzoesäure mit einer Reinheit von > 99,5 % gewonnen.

Das Ziel einer verminderten Korrosionswirkung von Reaktionsgemisch und Mutterlauge wird bei dem erfindungsgemäßen Verfahren durch den wäßrigen Verdünnungsgrad des Lösungsmittels im Reaktionsgemisch in Kombination mit einem, bezogen auf die Bromidionenkonzentration, größeren Gehalt in Grammäquivalent an Ammonium- und/oder Alkali- und/oder Erdalkaliionen erreicht. Dabei sind unter den Ammonium-, Alkali- oder Erdalkaliionen nicht ausschließlich die freien oder solvatisierten Ionen zu verstehen, sondern die Summe der sich in dem essigsauren Medium bei Zusatz beispielsweise der entsprechenden Acetate, Carbonate, Bromide oder Hydroxide bildenden Ammonium-, Alkali- oder Erdalkalikomponenten, unabhängig von dem Dissoziationsgrad.

Die stark korrosionsmindernde Wirkung einer Kombination von Wasser und Ammonium-, Alkali- oder Erdalkaliionen kann wie folgt gezeigt werden. So führte beispielsweise die Behandlung eines Blechstreifens aus Edelstahl der Werkstoff-Nr. 45 71 von 15 g mit einer geglätteten Oberfläche von 42 cm² (9,0 × 2,2 × 0,11 cm) mit einer Lösung von 4 g Kobaltbromid-hexahydrat in 600 g Essigsäure mit 0,5 Gew.-% Wasser nach 48 h bei 65 °C zu einem Gewichtsverlust des untersuchten Metalls von 3,5 g (23,3 Gew.-% bzw. 17,4 g/m² h). Bei Wiederholung dieses Versuches nach Zusatz von 3,0 g Natriumacetat und Erhöhung des Wassergehaltes auf 1 oder mehr Gew.-% wurde unter gleichen Versuchsbedingungen dagegen innerhalb der Meßgenauigkeit von 1 mg kein Gewichtsverlust festgestellt. Eine ähnlich positive Wirkung wurde auch durch Zusätze von Ammonium-, Kalium- oder Erdalkaliacetat bzw. diese in essigsaurer Lösung liefernde Substanzen wie Carbonaten oder Hydroxiden erzielt.

Die für das erfindungsgemäße Verfahren im Einsatzgemisch als notwendig angesehene Bromidionenkonzentration beträgt mindestens 0,0004 Äquivalente pro Mol Essigsäure. Das Reaktionsgemisch weist erfindungsgemäß zur Verminderung der Korrosivität pro Äquivalent vorhandener Bromionen und während der Durchführung des Verfahrens zusätzlich gebildeter Halogenionen 1,0 oder mehr Äquivalente und damit mehr als 0,000 4 Äquivalente pro Mol Essigsäure der Ammonium- und/oder Alkali- und/oder Erdalkalikomponente auf. In der Praxis wird ein deutlicher Überschuß der genannten Kationen von bis zu ca. 10 Äquivalenten pro Äquivalent im Einsatzgemisch vorgelegter Bromionen bevorzugt.

Die beim erfindungsgemäßen Verfahren bevorzugte Konzentration des als Cokatalysator eingesetzten Bromids beträgt 0,001 bis 0,01 Äquivalente pro Mol Essigsäure. Ein Teil des Bromids geht im Verlauf der Oxidationsreaktion verloren. Die Bromverluste werden bei Wiederverwendung der Mutterlauge durch entsprechende Zusätze ausgeglichen. Dazu wird vorzugsweise Bromwasserstoff in Form der wäßrigen oder essigsauren Lösung verwendet. Entsprechend der Bromidmenge im Einsatzgemisch beträgt die bevorzugte Ammonium-, Alkali- oder Erdalkalikonzentration ca. 0,002 bis 0,04 Äquivalente pro Mol Essigsäure.

Die Vorteile der Verfahrensweise unter Wiederverwendung der Mutterlauge werden im Falle der Herstellung der in Essigsäure relativ gut löslichen o-Chlorbenzoesäure besonders deutlich. Gemäß Beispiel 2 resultiert bei der Oxidation von o-Chlortoluol nach dem erfindungsgemäßen Verfahren zunächst nur eine Ausbeute an o-Chlorbenzoesäure von ca. 80 % d. Th. Bei Wiederverwendung der Mutterlauge wurde dann allerdings eine Ausbeute von > 97 % d. Th. realisiert. Dadurch steigt die Gesamtausbeute bereits nach einmaliger Wiederverwendung der Mutterlauge auf ca. 90 % d. Th. Nach 7maliger Rezyklisierung der Mutterlauge wird nach Beispiel 5 eine Gesamtausbeute von 94,8 % d. Th. an o-Chlorbenzoesäure erhalten, wobei das Produkt des letzten Versuches dieser Versuchsserie nach üblicher Aufarbeitung durch Abfiltrieren des Zielproduktes aus dem abgekühlten Reaktionsgemisch und Auswaschen des Filterkuchens mit einer wäßrig-verdünnten Essigsäure eine Reinheit von 99,9 % aufwies. Eine noch häufigere, die Gesamtausbeute weiter verbessernde Wiederverwendung der Mutterlauge erscheint damit durchaus möglich und sinnvoll.

Die Vorteile des erfindungsgemäßen Verfahrens betreffen jedoch nicht nur die technisch einfachere Produktisolierung, die Verringerung der Korrosionsprobleme und die durch eine mögliche Wiederverwendung der Mutterlauge bedingten Katalysatoreinsparungen. Überraschend erwies sich bei Anwendung der erfindungsgemäßen Alkalikonzentration, daß auch die Selektivität der Oxidation mit höheren als den im zitierten Stand der Technik angewandten Lösungsmittelmengen größer war. Dies wird durch einen Vergleich von Beispiel 6 mit dem Startversuch der im Beispiel 5 beschriebenen Versuchsserie der Oxidation von o-Chlortoluol deutlich.

Beispiel 5 und Beispiel 6 sind bezüglich der Einsatzmenge an o-Chlortoluol, Kobaltacetat, Natriumbromid und Natriumacetat identisch. Dies gilt auch für die Reaktions- und Aufarbeitungsbedingungen. Die Beispiele unterscheiden sich lediglich in Menge und Wassergehalt des Lösungsmittels während der Reaktion. Das Gewichtsverhältnis von Lösungsmittel zu o-Chlortoluol entspricht in Beispiel 6 mit 0,73 : 1 dem zitierten Stand der Technik, während das betreffende Verhältnis im Einsatzgemisch des unter den erfindungsgemäßen Bedingungen durchgeführten Beispiels 5 dagegen 2,45 : 1 betrug. Nach beendeter Oxidation wurden dann in Beispiel 6 durch Zusatz von Essigsäure und Wasser die gleichen Lösungsmittelverhältnisse wie in Beispiel 5 eingestellt. Nach identischer Aufarbeitung resultierte dann in Beispiel 5 mit 76,0 % d. Th. eine gegenüber Beispiel 6 mit 72,4 % d. Th. deutlich höhere Ausbeute an o-Chlorbenzoesäure.

Die Vorteile einer wäßrigen Verdünnung des Reaktionsmediums bezüglich der Ausbeute werden durch einen Vergleich der Beispiele 2 und 3 deutlich. Bei dem ohne Wasser im Einsatzgemisch durchgeführten Beispiel 3 wurde mit 71,9 % d. Th. nur eine sehr unbefriedigende Ausbeute an o-Chlorbenzoesäure erhalten. Unter ansonsten gleichen Reaktions- und Aufarbeitungsbedingungen ergab Beispiel 2 mit 0,3 Gew.-Teilen Wasser pro Gew.-Teil Essigsäure im Einsatzgemisch eine erheblich höhere Ausbeute von 80,7 % d. Th.

Beispiel 1

In einen mittels Ölumlauf beheizten und mit Rührer, Gaseinleitungsrohr, Temperaturfühler und Kondensator versehenen Autoklaven aus Hastelloy C wurden 300 g o-Chlortoluol, 660 g Essigsäure, 300 g Wasser, 15 g Kobaltacetattetrahydrat, 5 g Natriumbromid und 5 g Natriumacetat gefüllt und unter Stickstoff auf 150 °C erhitzt. Bei einer im Bereich 150 bis 155 °C gehaltenen Temperatur wurde dann Preßluft eingeleitet. Nachdem der Druck auf 25 bar gestiegen war, wurde die Gaseintrittsgeschwindigkeit durch die auf 3 l/min. eingestellte Gasaustrittsgeschwindigkeit des hinter dem Kondensator entspannten Abgases geregelt. Das Abgas wurde unter Verwendung entsprechender Meß- und Analysengeräte bezüglich Menge und Zusammensetzung, insbesondere auf den Sauerstoffgehalt hin, untersucht. Die Reaktionszeit betrug 210 min., wobei als Reaktionszeit die Zeitspanne vom Beginn der Lufteinleitung bis zur Beendigung der Sauerstoffaufnahme verstanden wird. Die Sauerstoffaufnahme wurde durch einen verminderten $O_2$-Gehalt im Abgas (< 21 %) gegenüber der eingeleiteten Preßluft erkannt. Aus dem nach beendeter $O_2$-Aufnahme auf Raumtemperatur abgekühlten Reaktionsgemisch wurde das Kristallisat durch Filtration isoliert, mit 450 g einer 50 Gew.-%igen Essigsäure gewaschen und getrocknet. Es resultierten 291,7 g o-Chlorbenzoesäure (78,6 % d. Th.) mit einer gaschromatographisch bestimmten Reinheit von 99,9 %.

Beispiel 2

Der in Beispiel 1 beschriebene Versuch wurde mit einer auf 200 g reduzierten Einsatzmenge Wasser bei einer Reaktionstemperatur von 140 bis 145 °C wiederholt. Dabei wurden 299,5 g o-Chlorbenzoesäure (80,7 % d. Th.) mit einer Reinheit von 99,9 % erhalten.

Die mit dem Waschfiltrat vereinigte Mutterlauge wurde unter Verwendung einer Fraktionierkolonne auf 700 g wasserfreie Lösung eingeengt. Eine Bromidbestimmung ergab einen Bromidgehalt von 0,038 Mol. Der sich daraus ergebende Bromidverlust von 22 % gegenüber der Einsatzmenge wurde durch 12 ml einer 7 %igen Bromwasserstoffsäure ausgeglichen, 190 g Wasser und 300 g o-Chlortoluol zugefügt und unter den zuvor genannten Bedingungen eine weitere Oxidation durchgeführt. Dabei resultierten 363,5 g o-Chlorbenzoesäure (97,9 % d. Th., bezogen auf frisch eingesetztes o-Chlortoluol) mit einer Reinheit von 99,9 %.

Beispiel 3 (Vergleichsbeispiel)

Der in Beispiel 1 beschriebene Versuch wurde ohne Wasser im Einsatzgemisch wiederholt. Nach einer Reaktionszeit von 200 min. bei 140 bis 145 °C und der üblichen Aufarbeitung wurden 293,7 g o-Chlorbenzoesäure (71,9 % d. Th.) gewonnen.

Beispiel 4

Der in Beispiel 1 beschriebene Versuch wurde mit auf 66 g Wasser und 6 g Kobaltacetat-tetrahydrat reduzierten Einsatzmengen bei einem Druck von 10 bar und einer Reaktionstemperatur von 150 °C wiederholt. Dabei wurden 282,1 g o-Chlorbenzoesäure (76,0 % d. Th.) erhalten.

Beispiel 5

Der in Beispiel 4 beschriebene Versuch wurde bei einem Druck von 25 bar wiederholt, wobei 282,2 g o-Chlorbenzoesäure resultierten. Entsprechend Beispiel 2 wurde die Mutterlauge auf 700 g wasserfreie Lösung eingeengt, die Bromidverluste (24,5 % des Einsatzes) durch 1,8 g einer 52 %igen Bromwasserstoffsäure ausgeglichen, 66 g Wasser und 300 g o-Chlortoluol zugegeben und eine weitere Oxidation unter gleichen Reaktionsbedingungen durchgeführt. Nach der üblichen Aufarbeitung wurden 368,7 g o-Chlorbenzoesäure (99,3 % d. Th. bezogen auf frisch eingesetztes o-Chlortoluol) mit einer Reinheit von 99,9 % gewonnen.

Die nach der zweiten Oxidation erhaltene und mit dem Waschfiltrat vereinigte Mutterlauge wurde wiederum in der beschriebenen Weise aufgearbeitet und zu einer Oxidation unter analogen Bedingungen eingesetzt. In gleicher Weise wurde dann noch weitere 5mal verfahren, wobei jeweils die entwässerte, auf 700 g eingeengte Mutterlauge mit 66 g Wasser, 300 g o-Chlortoluol und der zum Ausgleich der Bromidverluste erforderlichen Menge Bromwasserstoffsäure versetzt und einer erneuten Oxidationsreaktion unter stets gleichen Bedingungen unterworfen wurde.

Insgesamt wurden bei den 8 Versuchen dieser Versuchsserie 2 400 g o-Chlortoluol eingesetzt und 2 817 g Chlorbenzoesäure isoliert. Die Reinheit der im 8. Versuch gewonnenen o-Chlorbenzoesäure betrug 99,9 %. In der Mutterlauge des 8. Versuches wurden neben 0,019 Mol Bromid auch noch 0,045 Mol Chlorid nachgewiesen.

Beispiel 6 (Vergleichsbeispiel)

Der in Beispiel 1 beschriebene Versuch wurde bei einem Einsatz von 300 g o-Chlortoluol, 6 g Kobaltacetat-tetrahydrat, 5 g Natriumbromid, 5 g Natriumacetat und nur 210 g Essigsäure wiederholt. Nach beendeter Sauerstoffaufnahme wurden zunächst 450 g Essigsäure und 66 g Wasser zugefügt, bevor in der beschriebenen Weise aufgearbeitet wurde. Danach resultierten 276,4 g o-Chlorbenzoesäure (72,4 % d. Th.).

Beispiel 7

Der Startversuch der in Beispiel 5 beschriebenen Versuchsserie wurde mit einer auf 15 g erhöhten Einsatzmenge von Natriumacetat wiederholt und dabei 280 g o-Chlorbenzoesäure (75,5 % d. Th.) erhalten.

Beispiel 8

Entsprechend dem in Beispiel 1 erläuterten Verfahren wurde p-Chlortoluol zu p-Chlorbenzoesäure oxidiert. Das Einsatzgemisch bestand aus 250 g p-Chlortoluol, 700 g Essigsäure, 50 g Wasser, 3,5 g Kobaltbromid-hexahydrat, 3,5 g Kobaltacetat-tetrahydrat und 11 g einer 50 Gew.-%igen Kaliumhydroxid-Lösung. Die Oxidation wurde bei einem Druck von 25 bar, einem Gasaustritt von 2,5 l/min. und einer Temperatur von 150 bis 170 °C durchgeführt und ergab nach der üblichen Aufarbeitung 288 g p-Chlorbenzoesäure (90,5 % d. Th.) mit einer Reinheit von 99,9 %.

Beispiel 9

Entsprechend dem in den Beispielen 2 und 5 erläuterten Verfahren wurde 2,4-Dichlortoluol zu 2,4-Dichlorbenzoesäure oxidiert. Das Einsatzgemisch bestand aus 300 g 2,4-Dichlortoluol, 660 g Essigsäure, 73 g Wasser, 3 g Kobaltacetat-tetrahydrat, 6 g Manganacetat-tetrahydrat, 5 g Ammoniumbromid und 10 g Magnesiumacetat. Die Oxidation wurde unter den in Beispiel 1 beschriebenen Bedingungen durchgeführt und ergab nach der üblichen Aufarbeitung 303,5 g 2,4-Dichlorbenzoesäure (85,6 % d. Th.) mit einer Reinheit von 98,5 %.

Die analog Beispiel 2 auf 700 g wasserfreier Lösung eingeengte Mutterlauge mit einem Bromid- und Chloridgehalt von 0,25 bzw. 0,09 Gew.-% wurde nach Zusatz von 5,1 g einer 50 %igen Bromwasserstoffsäure, 73 g Wasser und 300 g Dichlortoluol einer erneuten Oxidationsreaktion unter gleichen Bedingungen unterworfen, wobei 337 g 2,4-Dichlorbenzoesäure (95,1 % d. Th.) resultierten.

Beispiel 10

Der in Beispiel 1 beschriebene Versuch wurde bei einem Einsatz von 300 g p-Fluortoluol, 660 g Essigsäure, 132 g Wasser, 6 g Kobaltacetat-tetrahydrat, 5 g Natriumbromid und 5 g Natriumacetat wiederholt. Bei einer Reaktionszeit von 240 min. wurden nach üblicher Aufarbeitung 330,5 g p-Fluorbenzoesäure (86,6 % d. Th.) mit einer Reinheit von 99,9 % erhalten.

Analog Beispiel 5 wurde die Mutterlauge weitere 3mal wiederverwendet. Dabei wurden jeweils 700 g wasserfreier Mutterlauge, nach Ausgleich der Bromidverluste durch Bromwasserstoffsäure, mit 122 g Wasser und 300 g p-Fluortoluol eingesetzt. Insgesamt wurden bei den 4 Versuchen 1 406,5 g p-Fluorbenzoesäure (92,1 % d. Th.) gewonnen.

### Beispiel 11

Der in Beispiel 10 beschriebene Versuch wurde unter Einsatz von 300 g p-Fluortoluol, 660 g Essigsäure, 35 g Wasser, 2 g Kobaltacetat-tetrahydrat, 4 g Manganacetattetrahydrat, 3 g Natriumbromid und 5 g Natriumacetat wiederholt. Nach beendeter Sauerstoffaufnahme wurde das heiße Reaktionsgemisch mit 450 g Wasser verdünnt und dann wie üblich aufgearbeitet. Dabei resultierten 344,5 g p-Fluorbenzoesäure (90,3 % d. Th.) mit einer Reinheit von 99,9 %.

700 g des destillativ aufgearbeiteten, wasserfreien Filtrates mit einem Bromidgehalt von 0,28 Gew.-% wurden mit 35 g Wasser, 0,7 g einer 50 %igen Bromwasserstoffsäure und 300 g p-Fluortoluol einer erneuten Oxidation unter gleichen Bedingungen zugeführt. Die Aufarbeitung erfolgte wiederum nach vorheriger Verdünnung des Reaktionsgemisches mit 450 g Wasser und ergab 363,5 g p-Fluorbenzoesäure (95,2 % d. Th.).

### Beispiel 12

Der in Beispiel 1 beschriebene Versuch wurde unter Einsatz von 300 g p-Bromtoluol, 700 g Essigsäure, 50 g Wasser, 6 g Kobaltacetat-tetrahydrat, 5 g Natriumbromid und 5 g Natriumacetat wiederholt. Bei einer Reaktionszeit von 170 min. wurden 292,4 g p-Brombenzoesäure (80 % d. Th.) erhalten.

### Beispiel 13

Der in Beispiel 1 beschriebene Versuch wurde unter Einsatz von 300 g o-Bromtoluol, 660 g Essigsäure, 60 g Wasser, 6 g Kobaltacetat-tetrahydrat, 5 g Natriumbromid und 5 g Natriumacetat wiederholt. Dabei resultierten nach einer Reaktionszeit von 155 min. 217 g o-Brombenzoesäure (61,6 % d. Th.).

Analog Beispiel 5 wurde die jeweils auf 700 g wasserfreier Lösung eingeengte Mutterlauge nach Zusatz von 60 g Wasser und 300 g bzw. 230 g o-Bromtoluol weitere 4mal zur Oxidation eingesetzt. Nach dem letzten nur mit 230 g o-Bromtoluol durchgeführten Versuch wurde das noch heiße Reaktionsgemisch vor der üblichen Aufarbeitung mit 300 g Wasser versetzt. Insgesamt wurden bei den 5 Versuchen 1 430 g o-Bromtoluol eingesetzt und 1 440 g o-Brombenzoesäure (85,7 % d. Th.) gewonnen.

### Patentansprüche

1. Verfahren zur Herstellung von Halogenbenzoesäuren durch Oxidation der entsprechenden, im Kern halogenierten Toluole, insbesondere von o-Chlortoluol, p-Chlortoluol, 2,4-Dichlortoluol, p-Fluortoluol, o-Bromtoluol oder p-Bromtoluol, mit Sauerstoff oder einem Sauerstoff enthaltenden Gas in Essigsäure als Lösungsmittel unter Zusatz löslicher Kobalt- und/oder Manganverbindungen und einer im Reaktionsgemisch eingesetzten Bromidionen liefernden Verbindung bei erhöhten Temperaturen von etwa 100 bis 180 °C und Drucken von etwa 1 bis 50 bar, dadurch gekennzeichnet, daß pro Gew.-Teil halogeniertem Toluol 1,5 bis 4,5 Gew.-Teile einer wässrig-verdünnten Essigsäure mit 1 bis 35 Gew.-% Wasser und einem Gehalt an einer löslichen Ammonium- und/oder Alkali- und/oder Erdalkaliverbindung von mehr als 1,0 Äquivalenten pro Äquivalent einer im Reaktionsgemisch eingesetzten, Bromidionen liefernden Verbindung, eingesetzt werden.

2. Verfahren nach Anspruch 1, dadurch gekennzeichnet, daß pro Äquivalent der Bromidionen liefernden Verbindung 1,2 bis 10 Äquivalente einer löslichen Ammonium-, Alkali- und/oder Erdalkaliverbindung in Form der Bromide, Acetate, Hydroxide bzw. Oxide, Carbonate oder Hydrogencarbonate eingesetzt werden.

### Claims

1. Process for the preparation of halobenzoic acids by oxidation of the corresponding nuclearly halogenated toluenes, in particular o-chlorotoluene, p-chlorotoluene, 2,4-dichlorotoluene, p-fluorotoluene, o-bromotoluene or p-bromotoluene, with oxygen or an oxygen-containing gas in acetic acid as solvent, with addition of soluble cobalt and/or manganese compounds and a compound supplying bromide ions employed in the reaction mixture, at elevated temperatures of about 100 to 180 °C and pressures of about 1 to 50 bar, characterised in that 1.5 to 4.5 parts by weight of a water-diluted acetic acid with 1 to 35 % by weight water and a content of a soluble ammonium and/or alkali and/or alkaline earth compound of more than 1.0 equivalents per equivalent of a compound supplying bromide ions employed in the reaction mixture are employed per part by weight of halogenated toluene.

2. Process according to claim 1, characterised in that 1.2 to 10 equivalents of a soluble ammonium, alkali and/or alkaline earth compound in the form of the bromides, acetates, hydroxides or oxides, carbonates or hydrogen carbonates are employed per equivalent of the compound supplying bromide ions.

**Revendications**

1. Procédé de préparation d'acides halogéno-benzoïques par oxydation des toluènes halogénés sur le noyau correspondant, en particulier d'ortho-chlorotoluène, para-chlorotoluène, 2,4-dichlorotoluène, para-fluorotoluène, orthobromo-toluène ou para-bromotoluène, avec de l'oxygène ou un gaz contenant de l'oxygène dans de l'acide acétique comme solvant avec addition de composés solubles de cobalt et/ou de manganèse et d'un composé fournissant des ions brome placés dans le mélange réactionnel, à des températures assez élevées d'environ 100 à 180 °C et sous des pressions d'environ de 1 à 50 bars, caractérisé en ce que pour une partie en poids de toluène halogéné, on emploie 1,5 à 4,5 parties en poids d'un acide acétique dilué à l'eau, contenant 1 à 35 % en poids d'eau et un composé soluble d'ammonium et/ou de métal alcalin et/ou de métal alcalino-terreux en quantité de plus de 1,0 équivalent par équivalent d'un composé fournissant des ions brome placés dans le mélange réactionnel.

2. Procédé selon la revendication 1, caractérisé en ce que pour un équivalent du composé fournissant des ions brome, on emploie 1,2 à 10 équivalents d'un composé soluble d'ammonium, de métal alcalin et/ou de métal alcalino-terreux sous forme de bromure d'acétate, d'hydroxyde ou d'oxyde, de carbonate ou d'hydrogénocarbonate.